# EUROPEAN PATENT APPLICATION

(11) **EP 0 995 797 A1**
(43) Date of publication of application: **26.04.2000**
(21) Application number: 98917705.0
(22) Date of filing: 27.04.1998
(51) Int. Cl.: C12N 15/09, C12Q 1/68

(54) **METHODS FOR DETECTING AND ISOLATING NUCLEAR TRANSPORT PROTEINS**

(30) Priority: 28.04.1997 JP 12479597; 24.10.1997 JP 30968697
(71) Applicant: Helix Research Institute, Chiba 292-0812 (JP)
(72) Inventor: UEKI, Nobuhide, Machida-shi, Tokyo 194-0003 (JP); NOGUCHI, Teruhisa, Fujisawa-shi, Kanagawa 251-0037 (JP)
(74) Representative: VOSSIUS & PARTNER
(86) International application number: JP9801936
(87) International publication number: WO9849284

(57) **Abstract**

Whether or not a test DNA encodes a peptide with nuclear transportability can be readily detected by introducing a fusion DNA formed by a DNA encoding a transcription factor whose nuclear transportability is eliminated and the test DNA into a eukaryotic host having in its nucleus a promoter region that is activated when said transcription factor binds thereto and a reporter gene whose expression is induced by said promoter region, and detecting the expression of said reporter gene. A DNA encoding a peptide with nuclear transportability is efficiently and systematically isolated by isolating a test DNA from cells expressing the reporter gene.

## Description

### Technical Field

The present invention relates to methods for detecting and isolating nuclear transport proteins and belongs to the field of genetic engineering, in particular, the field of gene cloning.

### Background Art

Various transcription factors, nuclear receptors, signal transduction factors, chromatin receptorsetc. are known as nuclear transport proteins. These proteins directly or indirectly interact with specific regions of DNA near the end of the intracellular signal transduction cascade, regulate gene expression, and replicate DNA to thereby determine the behavior of cells. Hence, to isolate the genes encoding these nuclear transport proteins and to analyze the function thereof is thought to be very important for clarifying various life phenomena or developing novel drugs.

However, special methods for systematically cloning cDNAs encoding nuclear transport proteins have not been developed, and so far, general methods applying cloning techniques have been used. In particular, a method in which a cDNA library is screened based on some information about the protein to be cloned, such as the protein having sequences conserved at the amino acid level (Lichtsteiner, S., Proc. Natl. Acad. Sci., 1993, 90: 9673-9677), the DNA sequence interacting with the protein (Sanz, L., Mol. Cell. Biol., 1995, 15: 3164-3170; MATCHMAKER One-Hybrid System (CLONTECH)), or proteins interacting with the protein to be cloned, has been employed when such information is available. In this case, screening has been possible only in extremely limited ranges.

For example, the two-hybrid system (Gyuris, J., Cell, 1993, 75: 791-803; Golemis, E. A., Current Protocols in Molecular Biology (John Wiley & Sons, Inc.), 1996, Ch. 20.0 and 20.1), which has recently been developed as a method for isolating proteins interacting with a protein, can indirectly screen the cDNAs encoding proteins interacting with a protein that has been known to coexist in the nucleus with said protein as a bait. However, it can not be used for directly screening the cDNAs encoding proteins with nuclear transportability. In addition, even if a protein known to exist in the nucleus is used as a bait (Jordan, K.L., Biochemistry, 1996, 35: 12320-12328), cDNAs encoding proteins other than nuclear proteins may be isolated because it is unknown whether the proteins interact in the cytoplasm before their transportation to the nucleus or whether they actually interact in the nucleus. This requires a laborious procedure to confirm whether the cDNAs isolated encode nuclear transport proteins or not. Besides, since the two-hybrid system uses the interaction between proteins as an indication, the proteins obtained through the screening are limited to those that can interact with the protein used as a bait.

When information about a desired protein as mentioned above is not obtained, the only method of screening a desired cDNA is to purify the protein from the nuclear fraction of cells using some function of the protein, such as specific bioactivity, and screening a cDNA library based on the sequence information of the protein obtained (Ostrowski, J., J. Biol. Chem., 1994, 269: 17626-17634). However, the purification of such proteins often takes much labor and time and, in some case, is substantially impossible since the expression level of some nuclear transport proteins is very low.

### Disclosure of the Invention

An objective of the present invention is to provide a method for easily and efficiently detecting and isolating a cDNA encoding a peptide having nuclear transportability.

An example of nuclear transport proteins is a transcription factor. Transcription factors of eukaryotes induce the expression of a specific gene by interacting with the promoter region of the said specific gene after their transportation to the nucleus. The nuclear transportability of a transcription factor is attributed to the nuclear transport signal existing in the transcription factor. The present inventors studied how to attain the objective mentioned above by focusing on two properties of transcription factors, the nuclear transportability and the ability activating the transcription of a specific gene. We thought that, when a fusion protein obtained by removing the region having nuclear transportability from a transcription factor and introducing an unknown peptide therefor is expressed, the fusion protein is transported into the nucleus and interacts with a specific promoter region to induce the expression of the specific gene downstream if said unknown peptide in the fusion protein has nuclear transportability. In contrast, if the unknown peptide in the fusion protein has no nuclear transportability, the fusion protein is not transported into the nucleus and does not induce the expression of the gene downstream of the specific promoter. Specifically, the present inventors thought that whether an unknown peptide in a fusion protein has nuclear transportability or not could be judged by observing if the fusion protein in which the unknown peptide is fused to a transcription factor without nuclear transportability induces the expression of the gene downstream of the specific promoter.

Based on such ideas, the present inventors prepared a fusion DNA formed by the DNA encoding a transcription factor from which the region having the nuclear transportability had been removed and the test DNA. We then introduced the fusion DNA into an eukaryotic host having in its nucleus a promoter region that is activated when a transcription factor binds thereto and a reporter gene whose expression is induced by the activation of said promoter region. Finally, we detected the expression of said reporter gene. As a result, the present inventors found that the expression of the reporter gene is induced when the test DNA encoding a peptide with nuclear transportability is used but not when the test DNA encoding a peptide without nuclear transportability is used.

The present inventors next prepared a library of cDNAs encoding fusion proteins formed by a transcription factor from which the region having the nuclear transportability had been removed and another peptide. We subsequently introduced the library into cells and screened cDNAs encoding peptides having the nuclear transportability using the expression of the reporter as an indication. As a result, the present inventors found that many of the known cDNAs among the cDNAs isolated from the cDNA library encode proteins that are thought to have nuclear transportability.

The present invention relates to a method for easily and efficiently detecting and isolating a DNA encoding a peptide having nuclear transportability using the properties of a transcription factor, and more specifically, to
(1) a method for detecting nuclear transportability of a peptide encoded by a test DNA, the method comprising introducing a fusion DNA formed by a DNA encoding a transcription factor without nuclear transportability and the test DNA into an eukaryotic host having in its nucleus a promoter region that is activated when said transcription factor binds thereto and a reporter gene connected to the downstream of said promoter region, and detecting expression of said reporter gene;
(2) the method of (1), wherein the transcription factor without nuclear transportability is a fusion protein comprising a nuclear export signal, a DNA binding domain, and a transcription activating domain;
(3) the method of (1), wherein the transcription factor without nuclear transportability is a fusion protein comprising a nuclear export signal, a LexA protein, and a GAL4-transcription activating domain, and the promoter region activated when said transcription factor binds thereto is that of a GAL1 gene whose operator sequence is replaced with that of LexA;
(4) the method of (3), wherein the nuclear export signal is a peptide comprising the amino acid sequence set forth in SEQ ID NO: 5;
(5) the method of any one of (1) to (4), wherein the reporter gene is LEU2 and/or a β-galactosidase gene;
(6) a method for isolating a DNA encoding a peptide with nuclear transportability, the method comprising introducing a fusion DNA formed by a DNA encoding a transcription factor without nuclear transportability and a test DNA into a eukaryotic host having in its nucleus a promoter region activated when said transcription factor binds thereto and a reporter gene connected downstream of said promoter region, detecting the expression of said reporter gene, and isolating the test DNA from the eukaryotic host in which the expression has been detected;
(7) the method of (6), wherein the transcription factor without nuclear transportability is a fusion protein comprising a nuclear export signal, a DNA binding domain, and a transcription activating domain;
(8) the method of (6), wherein the transcription factor without nuclear transportability is a fusion protein comprising a nuclear export signal, a LexA protein, and a GAL4-transcription activating domain, and the promoter region activated when said transcription factor binds thereto is that of a GAL1 gene whose operator sequence is replaced with that of LexA;
(9) the method of (8), wherein the nuclear export signal is a peptide comprising the amino acid sequence set forth in SEQ ID NO: 5;
(10) the method of any one of (6) to (9), wherein the reporter gene is LEU2 and/or a β-galactosidase gene,
(11) a vector comprising a DNA encoding a transcription factor without nuclear transportability and an introduction site for a test DNA adjacent thereto;
(12) the vector of (11), wherein the transcription factor without nuclear transportability is a fusion protein comprising a nuclear export signal, a DNA binding domain, and a transcription activating domain;
(13) the vector of (11), wherein the transcription factor without nuclear transportability is a fusion protein comprising a nuclear export signal, a LexA protein, and a GAL4-transcription activating domain;
(14) the vector of (13), wherein the nuclear export signal is the peptide comprising the amino acid sequence set forth in SEQ ID NO: 5;
(15) a kit comprising (i) a vector comprising a DNA encoding a transcription factor without nuclear transportability and an introduction site for a test DNA adjacent thereto, and (ii) a eukaryotic host having in its nucleus an expression unit comprising a promoter region activated when said transcription factor binds thereto and a reporter gene connected to the downstream of said promoter region;
(16) the kit of (15), wherein the transcription factor without nuclear transportability is a fusion protein comprising a nuclear export signal, a DNA binding domain, and a transcription activating domain;
(17) the method of (15), wherein the transcription factor without nuclear transportability is a fusion protein comprising a nuclear export signal, a LexA protein, and a GAL4-transcription activating domain, and the promoter region activated when said transcription factor binds thereto is that of a GAL1 gene whose operator sequence is replaced with that of LexA, and the eukaryotic host is yeast;
(18) the kit of (17), wherein the nuclear export signal is a peptide comprising the amino acid sequence set forth in SEQ ID NO: 5; and
(19) the kit of any one of (15) to (18), wherein the reporter gene is LEU2 and/or a β-galactosidase genes

The term "transcription factor" used herein means a protein that has a DNA binding domain and a transcription activating domain and that activates the transcription of a specific gene. It is not limited to a natural protein. The term "peptide" used herein includes not only a protein but also a partial peptide of a protein, a synthetic peptide, etc.

The first aspect of the present invention relates to a method for detecting nuclear transportability of a peptide encoded by a test DNA. The method comprises introducing a fusion DNA formed by a DNA encoding a transcription factor without nuclear transportability and the test DNA. This combination is then introduced into a eukaryotic host having in its nucleus a promoter region that is activated when said transcription factor binds thereto and a reporter gene whose expression is induced by the activation of said promoter region. The expression of said reporter gene is then detected.

The transcription factor used in the present invention to prepare a transcription factor without the nuclear transportability is not limited as long as the transcription factor can specifically regulate the expression of a gene in a eukaryote. Examples are GAL4 (Giniger, E., Cell, 1985, 40: 767-774), p53 (Chumakov, P.M., Genetika, 1988, 24: 602-612), GCN4 (Hinnenbusch, A. G., Proc. Natl. Acad. Sci., 1984, 81: 6442-6446), VP16 (Triezeneberg, S. J., Genes. Dev., 1988, 2: 718-729), RelA (Nolan, G.P., Cell, 1991, 64: 961-969), Oct-1 (Strum, R. A., Genes. Dev., 1988, 2: 1582-1599), c-Myc (Watt, R., Nature, 1983, 303: 725-728), c-Jun (Angel, P., Cell, 1988, 55: 875-885), and MyoD (Write, W. E., Cell, 1989, 56: 607-617).

The transcription factor without nuclear transportability in the present invention is not limited as long as it has transcription activating ability and DNA binding ability but no nuclear transportability (or extremely low nuclear transportability). Examples are a transcription factor whose nuclear transport signal is deleted or replaced with other amino acids, and a fusion protein comprising a DNA binding domain and a transcription activating region.

A nuclear pore is generally thought to be able to transfer low molecular weight substances (molecular weights 40 K Da or less) by diffusion as well as a specific active transport system. Even if the active nuclear transportability of a transcription factor is eliminated by deleting or replacing a nuclear transport signal, a substance can sometimes be transferred into the nucleus by diffusion. In this case, substance transfer into the nucleus by diffusion can be completely or minimally inhibited by further adding a signal localizing a protein in a cell other than the nucleus. The transcription factor without nuclear transportability of the present invention includes factors having a cell localization signal other than a nuclear localization signal. Examples of such a signal are a nuclear export signal (Gorlich, D., Science, 1996, 271: 1513-1518), a secretion signal, a peroxisome transport signal, a rough-surfaced endoplasmic reticulum transport signal, a mitochondrion transport signal (Nakai, K., Genomics, 1992, 14: 897-911; Nakai, K., PSORT WWW server, http://psort.nibb.ac.jp/), etc., but are not limited thereto.

In addition, there are transcription factors that have multiple nuclear transport signals or that have nuclear transportability but for which a nuclear transport signal site in the molecule has not been completely identified (GAL4, p53, etc. (Tanaka, M., Cell Science (in Japanese), 1991, 7: 265-272)). In addition, there are transcription factors whose DNA binding domains or transcription activating domains overlap with their nuclear transport signals and where deleting or replacing their nuclear transport signals is likely to eliminate their DNA binding ability or transcription activating ability. When such a transcription factor is used, even if it is impossible to completely identify the nuclear transport signal sequence, a transcription factor without nuclear transportability can be prepared by specifying the region essential for eliminating the nuclear transportability and deleting or replacing the region. In addition, a transcription factor without nuclear transportability can be prepared by generating an artificial hybrid transcription factor in which a DNA binding domain of a eukaryote- or prokaryote-derived protein that is known not to have a nuclear transport signal and a transcription activating domain that is known not to have a nuclear transport signal are fused. The "transcription factor without nuclear transportability" used herein includes the transcription factors thus prepared.

Examples of a transcription activating domain used herein to prepare a transcription factor without nuclear transportability include GAL4 (Brent, R., Cell, 1985, 43: 729-736), Bicoid, c-Fos, c-Myc, v-Myc, B6, B7, B42 (Golemis, A. E., Mol. Cell. Biol., 1992, 12: 3006-3014), GCN4 (Hope, I. A., Cell, 1986, 46: 885-894), and VP16 (CLONTECH, Mammalian MATCHMAKER Two-Hybrid Assay Kit), but are not limited thereto. In addition, examples of a DNA binding domain are those identified in transcription factors such as GAL4 (Giniger, E., Cell, 1985, 40: 767-774), p53 (Chumakov, P. M., Genetika, 1988, 24: 602-612), GCN4 (Hinnenbusch, A. G., Proc. Natl. Acad. Sci., 1984, 81: 6442-6446), VP16 (Triezeneberg, S. J., Genes. Dev., 1988, 2: 718-729), RelA (Nolan, G. P., Cell, 1991, 64: 961-969), Oct-1 (Strum, R. A., Genes. Dev., 1988, 2: 1582-1599), c-Myc (Watt, R., Nature, 1983, 303: 725-728), c-Jun (Angel, P., Cell, 1988, 55: 875-885), and MyoD (Write, W. E., Cell, 1989, 56: 607-617).

A DNA encoding a transcription factor without nuclear transportability can be prepared by a method in which the DNA sequence encoding the nuclear transport signal of the DNA encoding a transcription factor is completely or partially deleted, a method in which the sequence within the nuclear transport signal is replaced using site-directed mutagenesis, a method in which a cell localization signal other than a nuclear localization signal is added, a method in which a transcription activating domain is fused with a DNA biding domain, or a method in which these methods are appropriately combined. General gene manipulation in these methods is described in the literature (Sambrook, J., Molecular Cloning: A Laboratory Manual, 1989, 2nd Ed. Cold Spring Harbor Laboratory Press, Cold Spring Harbor, NY.).

The test DNA used herein is not limited as long as it encodes a protein or its partial peptide. The test DNA includes cDNA, genomic DNA, and synthetic DNA. A DNA encoding a transcription factor without nuclear transportability can be fused with a test DNA by the usual method (Sambrook, J., Molecular Cloning: A Laboratory Manual, 1989, 2nd Ed. Cold Spring Harbor Laboratory Press, Cold Spring Harbor, NY).

A fusion DNA formed by a DNA encoding a transcription factor without nuclear transportability and a test DNA is usually inserted into an appropriate expression vector, which is then introduced into a eukaryotic host. The expression vector is not limited as long as it can stably express in a eukaryotic host a protein encoded by a fusion DNA formed by a DNA encoding a transcription factor without nuclear transportability and a test DNA. A shuttle vector stably maintained in both a host and *E*. *coli* is preferable. For example, when baker's yeast is used as a host, a unit for expressing the protein is introduced into an integration vector that has no replication origin in it and that is integrated into the yeast chromosome, or into a plasmid vector that has a replication origin in it, that exists as a plasmid. (The expression unit here comprises a promoter region functioning in baker's yeast (e.g. that of ADH1 or GAL1), the coding region of an expressed protein, a multiple cloning site, and a terminator region (e.g. that of ADH1). For the plasmid vector, a centromere vector (low copy number), a 2 µ vector (high copy number), etc. are commercially available.) Specifically, for the integration vector and the centromere vector, pRS vector, which has various auxotrophy marker genes (LEU2, HIS3, URA3, TRP1, etc.) for complementing the host auxotrophy, is commercially available from STRATAGENE as a kit, which further comprises a mutant host strain corresponding to the respective marker genes. In addition, a marketed vector (HybriZapII, GAL4 Two-Hybrid Phagemid vector (STRATAGENE), MATCHMAKER vector (CLONTECH), etc.) that has various auxotrophy marker genes (LEU2, HIS3, URA3, TRP1, etc.) for complementing the host auxotrophy and that is used in a two-hybrid system, as well as a mutant host strain corresponding to the respective vectors, can be used for the 2 µ vector. When animal cells are used as hosts, a marketed and general mammalian expression vector, such as a vector that is integrated into the chromosome (pMAM, pMAM-neo (CLONTECH), etc.) or a vector maintained as an episome (λDR2, pDR2 vector system (CLONTECH), etc.), can be used with appropriate host animal cells (CHO, Mouse Fibroblast, Hela, U937, BHK, etc.). In addition, a vector for transient expression using COS cells, pMT2 (Sambrook, J., Molecular Cloning: A Laboratory Manual, 1989, 2nd Ed. Cold Spring Harbor Laboratory Press, Cold Spring Harbor, NY), can be used. The fusion DNA mentioned above can be inserted into an expression vector by the usual method (Sambrook, J., Molecular Cloning: A Laboratory Manual, 1989, 2nd Ed. Cold Spring Harbor Laboratory Press, Cold Spring Harbor, NY).

In addition, the eukaryotic host in the present invention into which the fusion DNA mentioned above is introduced is not limited as long as it can stably express the protein encoded by the fusion DNA. In particular, yeast and animal cultured cells are preferable from the viewpoints of simplicity in handling, ease of introducing and recovering a gene, safety, etc. The eukaryotic host used in this invention has in its nucleus a promoter region activated by binding of a specific transcription factor thereto, and a reporter gene connected to the downstream of the promoter region.

The promoter region activated by binding of a specific transcription factor thereto is not limited as long as the promoter region comprises a cis regulatory region called the upstream activating sequence (UAS) or operator sequence for binding the transcription factor and the TATA sequence and specifically activates transcription by binding the transcription factor to the UAS. Examples of the cis regulatory region of baker's yeast are natural GAL1 UAS (comprising four GAL4 binding sequences), artificial GAL1 UAS (comprising three GAL4 sequences), LexA UAS (comprising one to eight LexA binding sequences) (Estojak, J., Mole. Cell. Biol., 1995, 15: 5820-5829), etc. Examples of the TATA sequence are GAL1 TATA, CYS1 (cytochrome C1) TATA, LEU2 TATA, HIS3 TATA, etc. By combining the cis regulatory regions and the TATA sequences, various promoter regions whose expression level and induction condition differ from one another (CLONTECH, Yeast Protocols Handbook, PT3024-1: 5-8) can be constructed. Specifically, any promoter region that has a transcription factor binding sequence in the cis regulatory region and in which the transcription factor regulates the activity of the promoter can be used.

In addition, for baker's yeast, which has been thoroughly analyzed genetically, a reporter gene, such as a gene related to the auxotrophy of a host (LEU2, HIS3, TRP1, URA3, etc.), a gene (e.g. GAL1) related to the metabolism of an essential nutritive source, or a gene that can complement the deficiency of the other gene essential for survival enables easily detecting gene expression in terms of the viability of the host. Moreover, generally used reporters, such as a reporter gene whose expression can be detected by the enzymatic activity such as β-galactosidase, chloramphenicol acetyltransferase, or luciferase, and green fluorescent protein (CLONTECH) whose fluorescence can be directly detected leaving cells alive, are also available. In addition, the generally used reporter gene or the drug resistance gene mentioned previously can also be used in animal cells.

The promoter region and the reporter gene mentioned above can be connected by the usual method (Sambrook, J., Molecular Cloning: A Laboratory Manual, 1989, 2nd Ed. Cold Spring Harbor Laboratory Press, Cold Spring Harbor, NY).

A promoter region activated by binding of a specific transcription factor thereto and a reporter gene connected to the downstream of the promoter region can be introduced into, for example, baker's yeast used as a host by the usual method such as the lithium acetate method (CLONTECH, Yeast Protocols Handbook, PT3024-1: 17-20). A desired gene is integrated into a chromosome or allowed to exist as an intranuclear plasmid, depending on the difference in vectors used (the integration vector or the plasmid vector mentioned above). A gene can be introduced into animal cells by usual methods such as the liposome method (Sambrook, J., Molecular Cloning: A Laboratory Manual, 1989, 2nd Ed. Cold Spring Harbor Laboratory Press, Cold Spring Harbor, NY). A desired gene is integrated into a chromosome or allowed to remain as an intranuclear episome depending on the difference in vectors used (integration vector or episome vector mentioned above).

A commercial eukaryotic host organism having in its cell the promoter region and reporter gene mentioned above can be used. For example, when LexA is used as a DNA binding domain of a transcription factor, yeast EGY48[p8OP-lacZ] (available from CLONTECH), which has the promoter region containing the LexA operator sequence, and reporter genes LEU2 and β-galactosidase downstream thereof on both the chromosome and the plasmid can be used.

A vector comprising a fusion DNA formed by a DNA encoding a specific transcription factor without nuclear transportability and a test DNA can be introduced into a eukaryotic host, such as baker's yeast used as a host, by the usual method such as the lithium acetate method (CLONTECH, Yeast Protocols Handbook, PT3024-1: 17-20). A desired gene is integrated into a chromosome or is allowed to exist as an intranuclear plasmid depending on the difference in vectors used (the integration vector or the plasmid vector mentioned above). A gene can be introduced into animal cells by usual methods such as the liposome method (Sambrook, J., Molecular Cloning: A Laboratory Manual, 1989, 2nd Ed. Cold Spring Harbor Laboratory Press, Cold Spring Harbor, NY). A desired gene is integrated into a chromosome or is allowed to remain as an intranuclear episome depending on the difference in vectors used (the integration vector or the episome vector mentioned above).

The expression of a reporter gene in the transformants thus obtained can easily be detected based on the viability of the host, for example, baker's yeast, which has been subjected to thorough genetic analysis, when a gene (LEU2, HIS3, TRP1, URA3, etc.) related to the auxotrophy of the host, a gene (GAL1, etc.) related to the metabolism of an essential nutritive source, or a gene that can complement the deficiency of another gene essential for survival is used. In addition, β -galactosidase, chloramphenicol acetyltransferase, or luciferase, which is a generally used reporter, enables detecting the expression of a reporter gene based on the enzymatic activity, and green fluorescent protein (CLONTECH) enables directly detecting the fluorescence emitted by living cells. In animal cells, the expression of the generally used reporter gene or drug resistance gene mentioned previously can be detected similarly. A test DNA is thought to encode a peptide with nuclear transportability if the expression of the reporter gene is detected, but not if the expression of the reporter gene is not detected.

The second aspect of the present invention relates to a method for isolating a test DNA encoding a peptide with nuclear transportability. This method comprises introducing a fusion DNA formed by a DNA encoding a transcription factor without nuclear transportability and a test DNA into a eukaryotic host having in its nucleus a promoter region activated by binding of said transcription factor thereto and a reporter gene connected to the downstream of said promoter region, detecting the expression of said reporter gene, and isolating the test DNA from the eukaryotic host in which the expression has been detected. The test DNA can be isolated from the eukaryotic host, for example baker's yeast, in which the expression has been detected, by transforming *E. coli* with the plasmid isolated from a single colony and by isolating the plasmid again from said transformant when the test DNA exists on a plasmid (yeast-*E. coli* shuttle vector). Alternatively, the test DNA can be isolated by PCR amplification using the total DNA isolated from a single colony as a template (CLONTECH, Yeast Protocols Handbook, PT3024-1: 29-37). The test DNA can basically be isolated from animal cells by PCR amplification using the total DNA isolated from a single colony as a template.

A further aspect of the present invention relates to a vector comprising a DNA encoding a transcription factor without nuclear transportability and an introduction site for the test DNA adjacent thereto, and to a kit comprising said vector and a eukaryotic host. This eukaryotic host has in its nucleus an expression unit comprising a promoter region to which said transcription factor binds and a reporter gene connected to the downstream of said promoter region. After a test DNA is introduced into the introduction site for the test DNA, the vector of the present invention is introduced into the eukaryotic host having in its nucleus an expression unit composed of a promoter region to which said transcription factor binds and a reporter gene connected to the downstream of said promoter region. The introduction site for the test DNA is usually a site cleaved with a unique restriction enzyme on the vector. Introducing the vector into the eukaryotic host is thought to cause the test DNA introduced into the vector to encode a peptide with nuclear transportability if the expression of the reporter gene is detected in the eukaryotic host, but not if if the expression of the reporter gene is not detected. It is thus possible to easily detect whether the test DNA encodes a peptide with nuclear transportability and to easily isolate the DNA encoding a peptide with nuclear transportability. In particular, constructing a DNA library with the vector mentioned above, introducing the library into the eukaryotic host mentioned above, and detecting the expression of the reporter gene enables efficient and complete isolation of the DNA encoding a peptide with nuclear transportability from the library.

### Brief Description of the Drawings

Figure 1 shows the plasmid pLexAD.
Figure 2 shows the plasmid pLexADrev.
Figure 3 shows the plasmid pRS1F.
Figure 4 shows the plasmid pRS3F.
Figure 5 shows the assay for the nuclear transportability of the transcription factor used for fusing with a test peptide.
Figure 6 shows the assay for the nuclear transportability of the transcription factor fused with the test peptide.
Figure 7 shows the plasmid pNS.
Figure 8 shows the assay for the nuclear transportability of various peptides, using the plasmid pNS.

### Best Mode for Implementing the Invention

In the following, the present invention is explained in more detail with examples, but is not to be construed to be limited thereto. In the examples, the basic genetic engineering techniques followthe literature unless otherwise specified (Sambrook, J., Molecular Cloning: A Laboratory Manual, 1989, 2nd Ed. Cold Spring Harbor Laboratory Press, Cold Spring Harbor, NY). Products for genetic engineering such as restriction enzymes or other modifying enzymes were purchased from Takara Shuzo and used under the conditions described in the product manuals. QIAprep Kit (QIAGEN) was used to isolate a plasmid from *E. coli*. ABI PRISM 377 (PERKIN ELMER) was used to determine the nucleotide sequence. Reagents from the same company were used to prepare samples for analysis according to the product manuals. Yeast was manipulated using the MATCHMAKER LexA Two-Hybrid System (CLONTECH) (medium, host, shuttle vector, method for gene introduction, assay method for the reporter gene, method for gene isolation, etc.) following Yeast Protocols Handbook, which accompanied the system. In addition, synthesis of a custom oligonucleotide was ordered from Toa Gosei.

### Example 1 Construction of the nuclear transport protein trap vector

### (1) PCR amplification of the DNA sequence encoding GAL4 transcription activating domain

DNA fragments comprising the DNA sequence encoding the GAL4 transcription activating domain (the nucleotide sequence is shown in SEQ ID NO: 3) were amplified on GeneAmp PCR System 2400 (PERKIN ELMER) using primer NU13 (SEQ ID NO: 1), which has an add-in EcoRI site at its 5'-terminus, and MATCHMAKER 3' AD LD-Insert Screening Amplimer (CLONTECH) (SEQ ID NO: 2) as primers, and using plasmid pACT2 (CLONTECH) as a template. TaKaRa Ex Taq (TaKaRa) was used as Taq polymerase under the conditions described in the product manual. The DNA fragments thus amplified were purified by ethanol precipitation and digested with restriction enzymes EcoRI and NcoI, then subjected to polyacrylamide gel electrophoresis using 6% polyacrylamide gel. The desired DNA fragment was then cut out of the gel and recovered by the electroelution method.

### (2) construction of the vector pLexAD expressing the fusion protein formed by the LexA protein and the GAL4 transcription activating domain

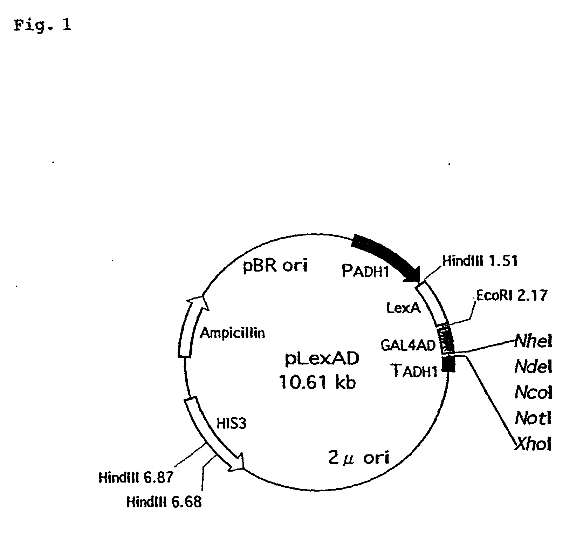

pLexAD was constructed by inserting the DNA fragment encoding the GAL4 transcription activating domain of (1) above between the EcoRI and NcoI sites within the multiple cloning site of plasmid pLexA (CLONTECH) (Fig. 1). Nucleotide sequence determination confirmed that the desired fragment was correctly inserted. The nucleotide sequence of the LexA gene is shown in SEQ ID NO: 4.

### (3) Construction of the vector pLexADrev, in which the nuclear export signal (NES) is inserted into the N terminus of LexA

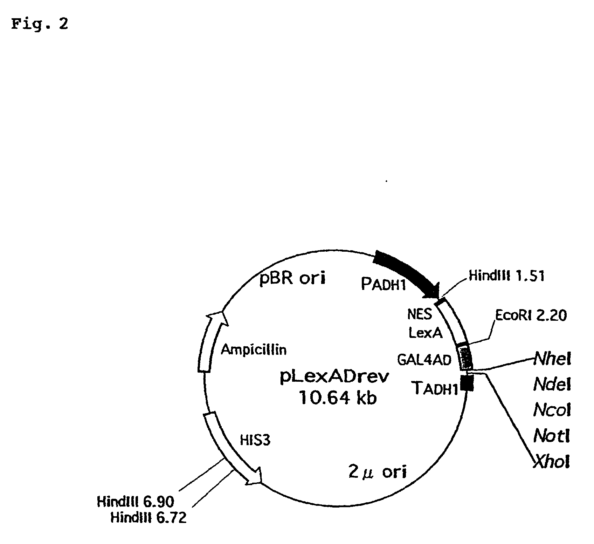

The nuclear export signal (SEQ ID NO: 5) of the Rev protein of HIV was synthesized as follows and inserted into the HpaI site near the N terminus of the LexA protein encoded by pLexAD. NU9 (SEQ ID NO: 6) and NU10 (SEQ ID NO: 7) were synthesized as the sense and antisense strands, respectively; phosphorylated at their 5' termini with T4 polynucleotide kinase; and annealed with each other. This DNA fragment was digested with HpaI and inserted into pLexAD that had been dephosphorylated with alkaline phosphatase, to construct pLexADrev (Fig. 2). Nucleotide sequence determination confirmed that the desired fragment was properly inserted.

### (4) Construction of plasmid pRS1F having at its replication origin the CEN/ARS region for expressing the fusion protein formed by the LexA protein and the GAL4 transcription activating domain, and plasmid pRS3F having at its replication origin the CEN/ARS region for expressing the fusion protein formed by the LexA protein into which NES is inserted and the GAL4 transcription activating domain

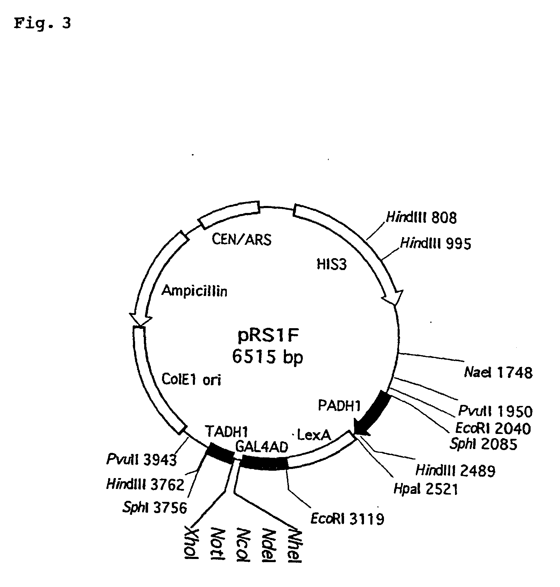

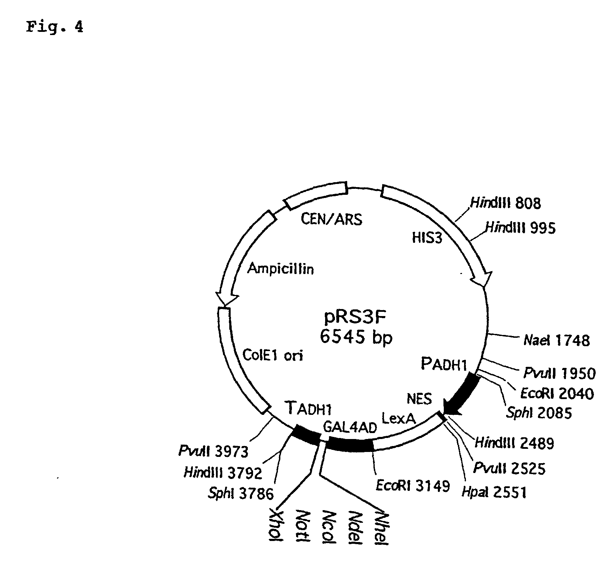

The minimal unit for expressing in yeast the fusion protein formed by the usual LexA protein without the nuclear export signal (NES) and the GAL4 transcription activating domain is the DNA fragment of about 1.7 kb obtained by digesting pLexAD with SphI. The minimal unit for expressing in yeast the fusion protein formed by the LexA protein into which NES is inserted at its N terminus and the GAL4 transcription activating domain (the nucleotide sequence described with the amino acid sequence of the fusion protein is shown in SEQ ID NO: 8) is the DNA fragment of about 1.7 kb obtained by digesting pLexADrev with SphI. This expression unit comprises the ADH1 promoter region, the coding region of a protein to be expressed, the multiple cloning site, and the ADH1 terminator region. After being purified, the DNA fragments of the respective expression units were inserted into the SphI site of the vector pRSF. The SphI site was constructed by replacing the PvuII fragment comprising the multiple cloning site of plasmid pRS413 (STRATAGENE) (yeast shuttle vector, CEN/ARS origin) with the PvuII fragment comprising the multiple cloning site of the generally used plasmid pUC19, to construct pRS1F and pRS3F (Figures 3 and 4, respectively). Nucleotide sequence determination confirmed that the desired fragment was properly inserted. Since pRS1F (positive control) and pRS3F thus constructed have the pLexA-derived multiple cloning site immediately after the gene encoding a fusion protein functioning as a transcription factor, a DNA fragment such as the desired cDNA can easily be fused and expressed by the usual method.

### Example 2 Demonstration of effectiveness of the nuclear transport protein trap vector pRS3F by fusing the cDNA of an artificial nuclear transport protein

### (1) Fusing known cDNA fragments

The known cDNA fragment, *Pseudomonas aeruginosa* branched-chain-amino-acid binding protein ('BraC), is localized in cytoplasm whose secretion signal had been deleted (the nucleotide sequence described with the amino acid sequence of said protein is shown in SEQ ID NO: 9) (Tanaka, M., New Lectures on Biochemical Experiments, Vol. 6, Ed. Japanese Biochemical Society, Biomembrane and Membrane Transport, Vol. 2, 1992, Tokyo Kagaku Dojin, 9·15). This known cDNA fragment was fused at its N terminus with the nuclear transport signal derived from SV40 large T antigen to construct a cDNA fragment encoding an artificial nuclear transport protein. This fragment was then fused in-frame to the C terminus of the GAL4 transcription activating domain of pRS3F. Specifically, the DNA fragment (NcoI-DraI) encoding 'BraC was inserted into pRS3F, which had been digested with XhoI, blunted with Klenow treatment, digested with NcoI, and purified to construct pRS3F'BraC. Moreover, the synthetic DNA fragment encoding the nuclear transport signal derived from SV40 large T antigen (SEQ ID NO: 10) was inserted into pRS3F'BraC that had been digested with NheI and NcoI and purified to construct pRS3FN'BraC. The synthetic DNA fragment was prepared in such a way that NU17 (SEQ ID NO: 11) and NU18 (SEQ ID NO: 12) were synthesized as the sense and antisense strands, respectively; phosphorylated at their 5' termini with T4 polynucleotide kinase; and annealed with each other. As control, pRS3FN, which had the nuclear transport signal but not the 'BraC fragment, was constructed similarly. Nucleotide sequence determination confirmed that the desired fragment was properly inserted.

### (2) Assay for nuclear transportability by the expression of a reporter gene

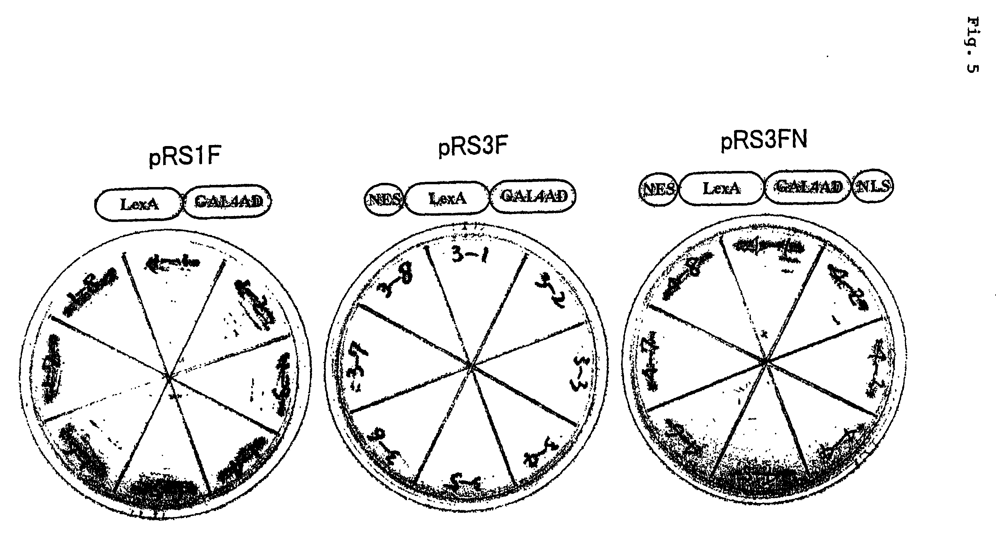

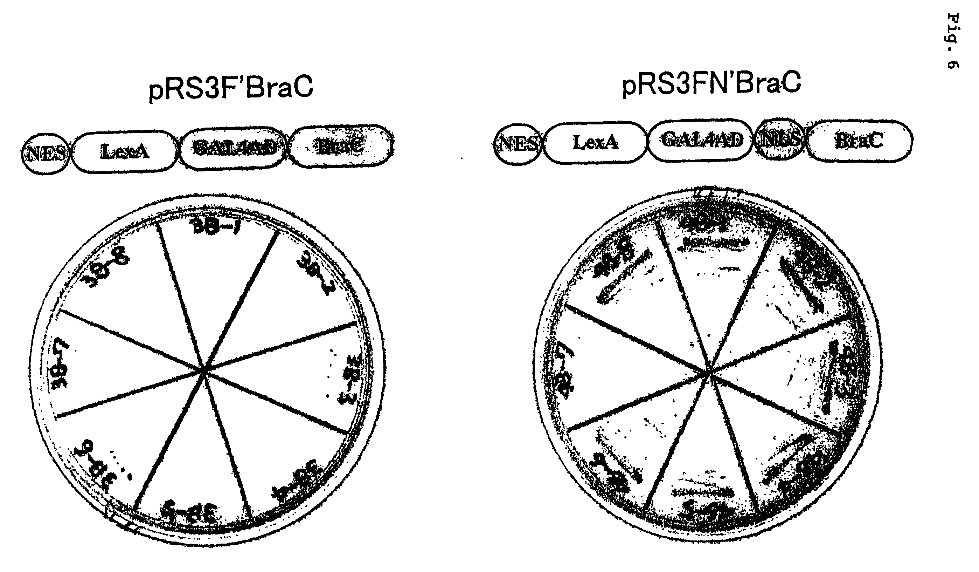

Host yeast EGY48[p8OP-lacZ] (CLONTECH), which has the promoter region containing the LexA operator sequence (SEQ ID NO: 13) (Estojak, J. Mole. Cell. Biol., 1995, 15: 5820-5829), and reporter genes LEU2 and β-galactosidase downstream thereof on both the chromosome and plasmid, was transformed with the three plasmids pRS3F'BraC, pRS3FN'BraC, and pRS3FN or the plasmids constructed in Example 1, pRS1F and pRS3F. Introduction of the desired plasmid into hosts was confirmed by the complementarity of HIS, an auxotrophy marker. Each of the transformants was then replicated in a medium (SD/-LEU, -HIS, -URA, X-gal) to assay the expression of the reporter genes and cultivated at 30°C for two to three days. As a result, reporter genes β-galactosidase and LEU2 were both expressed in the transformants carrying pRS3FN'BraC in which the artificial nuclear transport protein had been fused, pRS3FN in which only the nuclear transport signal had been fused, or pRS1F, a positive control, and both blue coloring and normal growth were confirmed (Figs. 5 and 6). In contrast, the reporter genes were hardly expressed in the transformants carrying pRS3F'BraC in which the protein without the nuclear transport signal had been fused, and pRS3F in which nothing had been fused, and neither blue coloring nor normal growth were observed (Figs. 5 and 6).

The above results indicate that whether a DNA fragment encoding a peptide has nuclear transportability or not could be easily determined by fusing the DNA fragment in-frame to the C terminus of the transcription factor encoded by pRS3F, expressing the fusion protein in yeast, and detecting the expression of the reporter gene as an indication.

### Example 3 Construction of the vector pNS for preparing a cDNA library

pRS3F was modified as follows. (1) The EcoRI site at the junction between LexA and GAL4AD was removed. (2) A new EcoRI site was introduced into the multiple cloning site. (3) The unnecessary region derived from pRS413 was removed to minimize the size of the vector.

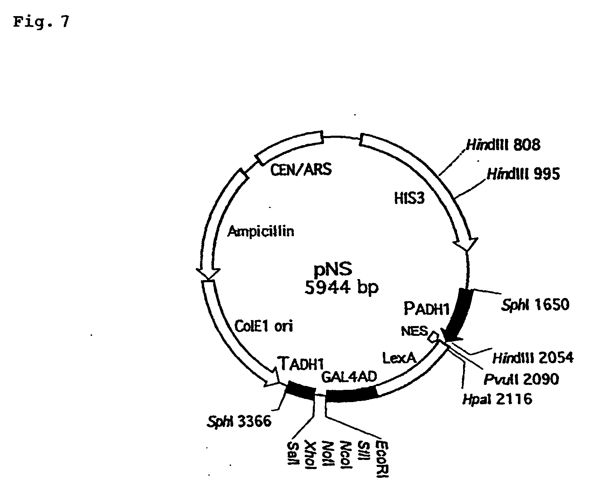

First, a synthetic linker, with sense strand NU31 (SEQ ID NO: 14) and antisense strand NU30 (SEQ ID NO: 15), was inserted into the EcoRI site of pLexADrev to obtain the plasmid pLexADrev-dE. The DNA fragment of about 1.7 kb containing the ADH1 expression unit and obtained by digesting pLexADrev-dE with a restriction enzyme SphI was subcloned into the SphI site of a generally used plasmid pUC19 to obtain the plasmid pULexADrev-dE. A synthetic linker with an EcoRI site and with sense strand NU28 (SEQ ID NO: 16) and antisense strand NU29 (SEQ ID NO: 17), was inserted between the NheI site and the NcoI site of pULexADrev-dE to obtain the plasmid pULexADrev-E. pRS413 was digested with DraIII and PvuII to remove a 757 bp DNA fragment comprising the multiple cloning site. A synthetic linker with an SphI site, whose sense strand was NU25 (SEQ ID NO: 18) and antisense strands was NU26 (SEQ ID NO: 19) was inserted into the digested plasmid to obtain the plasmid pRS-S. The DNA fragment of about 1.7 kb containing the ADH1 expression unit and obtained by digesting the above-mentioned plasmid pULexADrev-E with SphI was inserted into the SphI site of pRS-S to construct the vector pNS for preparing a cDNA library (Fig. 7). (The transcription direction by ADH1 is the same as that of HIS3.)

### Example 4 Construction of the fusion protein-expression library (derived from human cultured cells, NT2 precursor cells) and the nuclear transport assay

### (1) Construction of the fusion protein-expression library

Human cultured cells (NT2 precursor cells (Stratagene)) were cultivated following the recommended protocol (Catalog #204101, Revision #036002a), and the mRNA was prepared using the commercial total mRNA extraction kit and the mRNA extraction kit (Pharmacia). A cDNA library was constructed using a 3 µgportion of the mRNA obtained above and the commercial cDNA synthetic kit (Pharmacia). Specifically, cDNA was synthesized using oligo(dT)12-18 primer and inserted into the EcoRI/NotI sites of the pNS vector. The cDNA was unidirectionally inserted using the Directional Cloning Toolbox (Pharmacia). Commercial *E. coli* (ElectroMAX DH10B Cells from GibcoBRL) was transformed with a part of the cDNA library thus constructed by electroporation (Gene Pulser from BIO RAD) according to the usual method (New Protocols for Cell Engineering Experiments, Shujunsha, 114-115). The transformants obtained were cultivated on LB agar media comprising ampicillin (100 µ g/ml) at 30 °C for 16 hours. After harvesting, the plasmids were prepared (QIAGEN Maxi kit from QIAGEN).

### (2) Nuclear transport assay using yeast

EGY48 was transformed with 60 µg of the plasmid of the fusion protein-expression library prepared above by the usual method (CLONTECH, Yeast Protocols Handbook, PT3024-1: 17-20). The transformants were cultivated on SD agar media (-His/-Leu) at 30°C for three to seven days to screen the clones for expression of reporter gene LEU2, and about 1,000 positive clones were obtained.

### (3) Nucleotide sequencing

The nucleotide sequence of the cDNA fragment inserted into the vector was determined with 12 of the positive clones so obtained. To determine the nucleotide sequence, the template DNA was first prepared from each clone by colony PCR. A small amount of bacterial cells harvested from each clone was added to 20 µl of a PCR reaction mixture (0.5 units of thermotolerant DNA polymerase (Ex Taq from TaKaRa), 4 nmol of dNTP mixture, 0.4 pmol each of primer NU15 (SEQ ID NO: 20) and primer NU36 (SEQ ID NO: 21), 2 µl of the attached buffer, and sterilized water). The inserted cDNA fragment was then amplified on GeneAmp PCR System 2400 (PERKIN ELMER) for 40 cycles of incubation at 94°C for denaturation, incubation at 60°C for annealing, and incubation at 72°C for extension. Unreacted primers were desalted and removed from each PCR product using Microcon-100 (Millipore) to obtain the template DNA. A 100 to 200 ng portion of the template so obtained was sequenced by the method recommended in the product manual of ABI.

### (4) Database analysis of the obtained clones

The nucleotide sequence of each clone was searched in a public database, Basic BLAST (http://www.ncbi.nlm.nih.gov/cgi-bin/BLAST/nph-blast?Jform=0) of the National Center for Biotechnology Information (NCBI). As a result, all 12 clones were found to coincide with known genes. It has been so far reported or suggested that 10 of the 12 clones function in the nuclei. Of those 10, five clones, NP220 (Inagaki, H., J. Biol. Chem., 1996, 271: 12525-12531), PC4 (Ge, H., Cell, 1994, 78: 513-523), ERC-55 (Imai, T., Biochem. Biophys. Res. Commun., 1997, 233: 765-769), histone binding protein (O'Rand, M. G., Dev. Biol., 1992, 154: 37-44), and prothymosin α1 (Manrow, R. E., J. Biol. Chem., 1991, 266: 3916-3924), have sequences rich in basic amino acids similar to the nuclear transport signal of SV40 large T antigen type. Furthermore, one clone, hnRNPA1 (Michael, W. M., Cell, 1995, 83: 415-422), has the M9 sequence, which plays a role in both nuclear import and export, and four clones, ferritin H chain (Cai, C. X., J. Biol. Chem., 1997, 272: 12831-12839), chaperonine 10 (Bonardi, M. A., Biochem. Biophys. Res. Commun., 1995, 206: 260-265), protein kinase C inhibitor-I (Brzoska, P. M., Proc. Natl. Acad. Sci., 1995, 92: 7824-7828), and steroid receptor co-activator 1 (Onate, S. A., Science, 1995, 270: 1354-1357), have no known nuclear transport signal. No known nuclear transport signal was found in the remaining two clones, tropomyosin (Lin, C.-S., Mol. Cell. Biol., 1988, 8: 160-168) and G-rich sequence factor-1 (Qian, Z., Nucleic Acids Res., 1994, 22: 2334-2343), whose function in the nuclei had not been reported to date.

### Example 5 Construction of the fusion protein-expression library (derived from human fetal brain) and nuclear transport assay

### (1) construction of the fusion protein-expression library

First, the commercial human fetal brain cDNA library (SUPERSCRIPT library from GibcoBRL) was amplified according to the recommended protocol. The plasmid having the cDNA insert was then purified using the plasmid isolation kit from QIAGEN. Next, the cDNA fragments excised from the portion thereof (30 µg) with two kinds of restriction enzymes, EcoRI and NotI, were grouped by size within 0.7 to 4 kb and purified by 0.8% agarose gel electrophoresis. The cDNA fragments so obtained were inserted into the EcoRI/NotI sites of the pNS vector mentioned above to construct the fusion protein-expression library. The commercial *E. coli* (ElectroMAX DH10B Cells from GibcoBRL) was transformed with the portion of the library thus obtained by electroporation (Gene Pulser from BIO RAD) according to the usual method (New Protocols for Cell Engineering Experiments, Shujunsha, 114-115). The resulting transformants were cultivated on LB agar media comprising ampicillin (100 µg/ml) at 30°C for 16 hours. After harvesting, the plasmids were prepared (QIAGEN Maxi kit from QIAGEN).

### (2) Nuclear transport assay using yeast

EGY48 was transformed with 60 µg of the plasmid of the fusion protein-expression library prepared by the usual method (CLONTECH, Yeast protocols Handbook, PT3024-1: 17-20). The transformants were cultivated on SD agar media (-His/-Leu) at 30°C for three to seven days to screen the clones for expression of the reporter gene LEU2, and about 1,000 positive clones were obtained.

### (3) Nucleotide sequence determination

The nucleotide sequence of the cDNA fragment inserted into the vector was determined with 489 of the positive clones so obtained. To determine the nucleotide sequence, the template DNA was first prepared from each clone by colony PCR. A small amount of bacterial cells harvested from each clone was added to 20 µ l of a PCR reaction mixture (0.5 unit of thermotolerant DNA polymerase (Ex Taq from TaKaRa), 4 nmol of the dNTP mixture, 0.4 pmol each of primer NU15 (SEQ ID NO: 22) and primer NU36 (SEQ ID NO: 23), 2 µl of the attached buffer, and sterilized water). The cDNA insert was then amplified on GeneAmp PCR System 9600 (PERKIN ELMER) for 40 cycles of incubation at 94°C for denaturation, incubation at 60°C for annealing, and incubation at 72°C for extension. Unreacted primers were desalted and removed from each PCR product using a Microcon-100 (Millipore) to obtain the template DNA. A 100 to 200 ng portion of the template so obtained was subjected to nucleotide sequence determination by the method according to the product manual of ABI.

### (4) Database analysis of the obtained clones

The nucleotide sequences of the 489 clones were searched in a public database, Basic BLAST (http://www.ncbi.nlm.nih.gov/cgi-bin/BLAST/nph-blast?Jform=0) of the National Center for Biotechnology Information (NCBI). As a result, 250 clones coincided with the 97 genes encoding known proteins (Tables 1 and 2), and 220 clones coincided with the 172 genes encoding novel sequences or known Expressed Sequence Tag (EST) sequences as candidates for genes encoding novel nuclear transport proteins. In 19 clones, the sequences were derived from the non-coding regions of known genes or the reading frames of the codons were shifted.

Of the genes isolated by the method of the present invention, Table 1 shows the genes encoding proteins reported to function in the nuclei and Table 2 shows the genes not reported to function in the nuclei.

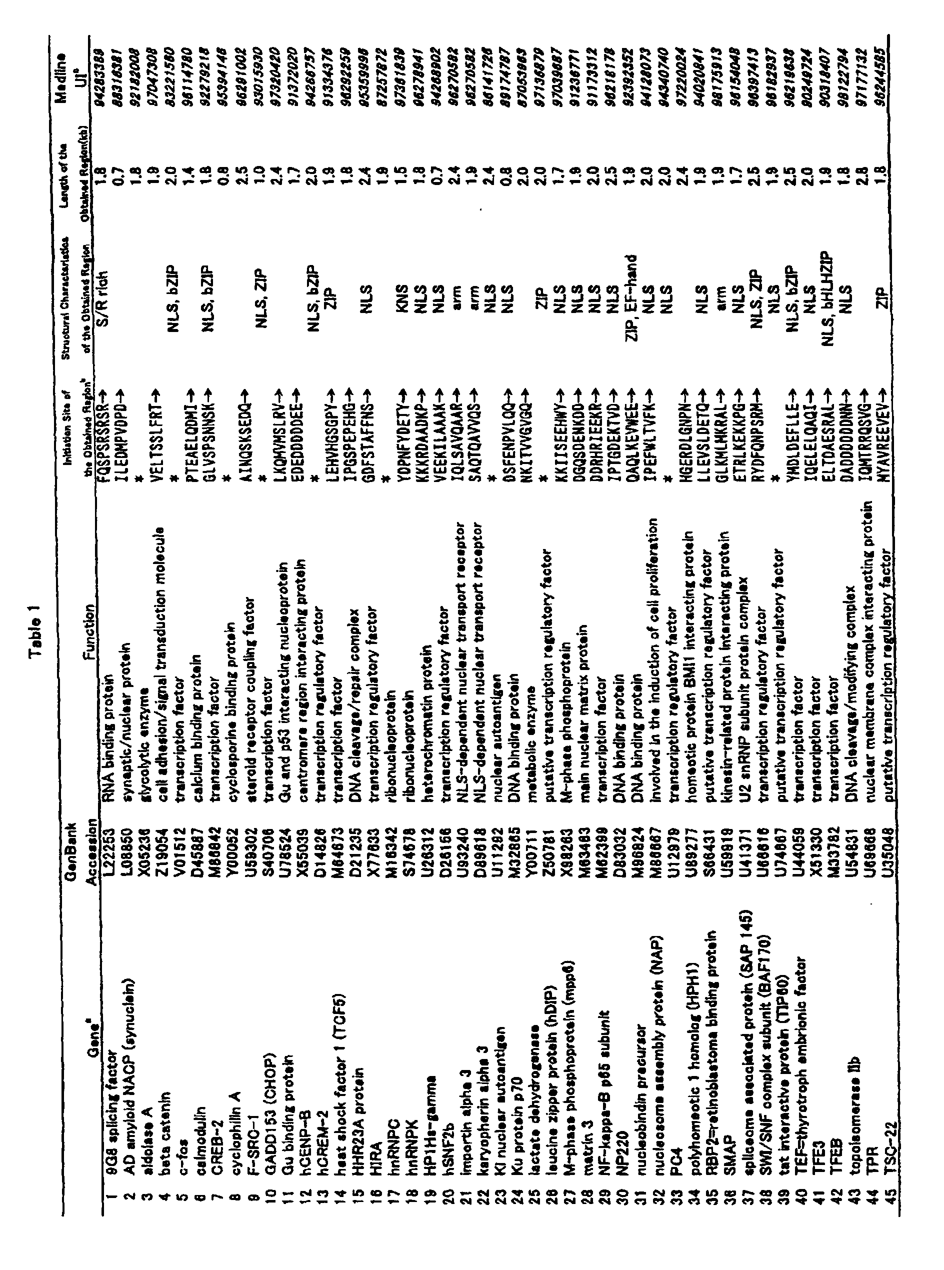

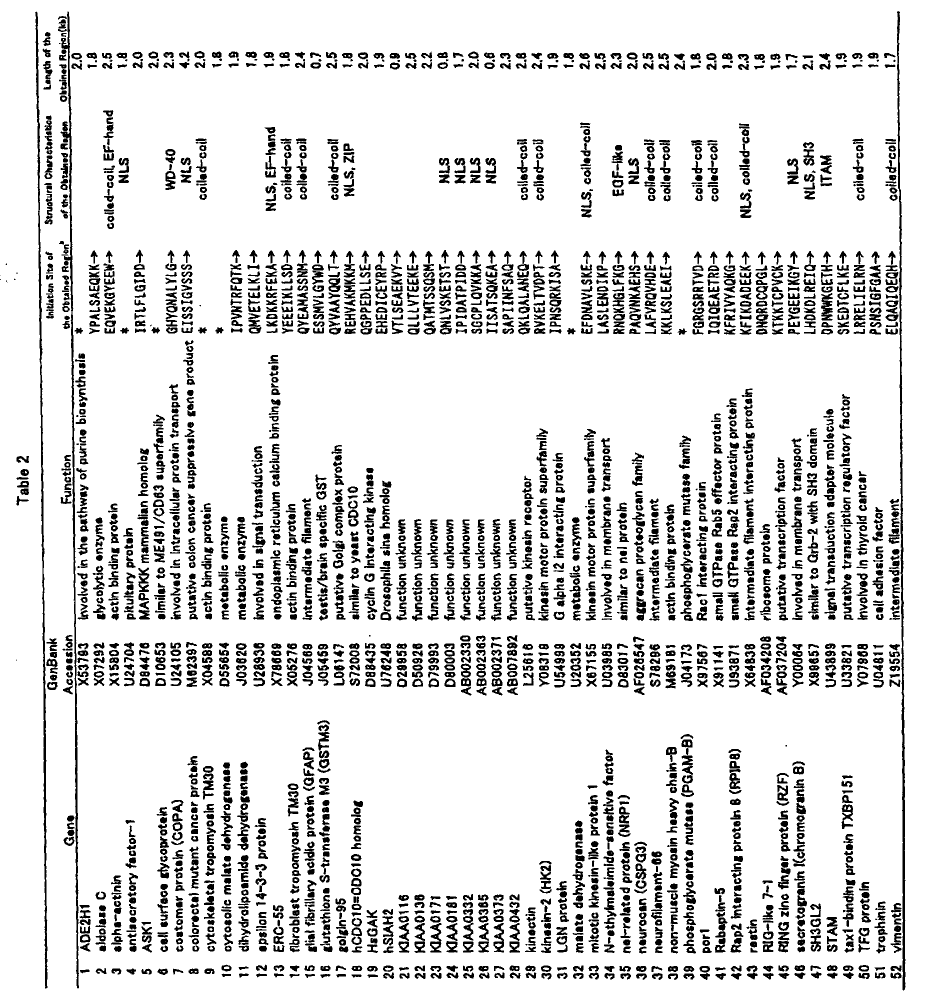

The symbols in the Tables are defined as follows.
^{a} The shortest insert among the obtained genes grouped as a representative.
^{b} Ten amino acids from the N terminus of the protein encoded by the gene insert.
^{c} Medline Unique Identifier of the literature reporting the function in the nucleus
* Clone comprising the whole coding region

S/R rich, region rich in serine/arginine; NLS, putative nuclear transport signal rich in basic amino acid residues; ZIP, leucine zipper; bZIP, basic leucine zipper; KNS, hnRNP K nuclear transport signal; arm, armadillo repeat; bHLHZIP, basic helix-loop-helix leucine zipper; SH3 Src homology domain 3; ITAM, immunoreceptor tyrosine-based activation motif

As shown in Tables 1 and 2, at least half of the 97 known proteins are reported to function in the nuclei. In particular, many transcription factors and DNA/RNA binding proteins are included. Hence, novel genes encoding unknown proteins that function in the nuclei have been efficiently and specifically obtained. In addition, KNS sequence (Matthew, W., EMBO J., 1997, 16: 3587-3598), which plays a role in both nuclear import and export, has been found in the hnRNP K protein of the isolated clones. The fact that the M9 sequence and the KNS sequence, which play a role in both nuclear import and export, have been found in the clones isolated by the method of the present invention demonstrates not only that the present invention can efficiently and specifically screen nuclear transport proteins but also that it can be expanded to screen nuclear import and export proteins (both import into the nucleus and export from the nucleus).

### Example 6 Demonstration of the effectiveness of the nuclear transport protein trap vector pNS by fusing the cDNA encoding a known nuclear transport protein

### (1) Construction of a fusion plasmid of known cDNA fragments

The cDNAs of 'BraC (Tanaka, M., New Lectures on Biochemical Experiments, Vol. 6, Ed. Japanese Biochemical Society, Biomembrane and Membrane Transport, Vol. 2, 1992, Tokyo Kagaku Dojin, 9·15) and Ca²⁺/calmodulin-dependent protein kinase CaMKK (Tokumitsu, H., J. Biol. Chem., 1995, 270(33): 19320-19324; Tokumitsu, H., Intracellular localization of CaMKK, unpublished data) were used as representative proteins localized in the cytoplasm. In addition, the cDNAs of NLS of SV40, NLS-'BraC, in which NLS of SV40 and 'BraC were artificially fused; transcription factor NF-kappa-B p65 subunit NFKBp65 (Ganchi, P. A., Mol. Biol. Cell, 1992, 3(12): 1339-1352);and transcription factor c-Fos (Tratner, I., Oncogene, 1991, 6(11): 2049-2053) were used as representative proteins localized in the nuclei and having the typical nuclear transport signal. The plasmid pRS1F was used for LexAD, pNS for NES-LexAD, pRS3FN for NES-LexAD-NLS, pRS3F'BraC for NES-LexAD-'BraC, and pRS3FN'BraC for NES-LexAD-NLS-'BraC. After NFKBp65 was amplified by PCR using pME18S(N)-p65 (Tsuboi, A., Biochem. Biophys. Res. Commun., 1994, 199(2): 1064-1072) as a template and NU32 (SEQ ID NO: 24) and NU24 (SEQ ID NO: 25) as primers, the fragment was digested with restriction enzymes MunI and NotI, and inserted into the EcoRI/NotI sites of pNS to construct NES-LexAD-NFKBp65. Similarly, after c-Fos was amplified by PCR using pME18S(N)-c-Fos (Tsuboi, A., Biochem. Biophys. Res. Commun., 1994, 199(2): 1064-1072) as a template and NU34 (SEQ ID NO: 26) and NU24 as primers, the fragment was inserted into the EcoRI/NotI site of pNS to construct NES-LexAD-cFos. The CaMKK cDNA fragment generated by digesting pET-CaMKK (gift from H. Tokumitsu) with the restriction enzyme NcoI was inserted into the NcoI site of pNS to construct NES-LexAD-CaMKK.

### (2) Detection of nuclear transportability by the expression of a reporter gene

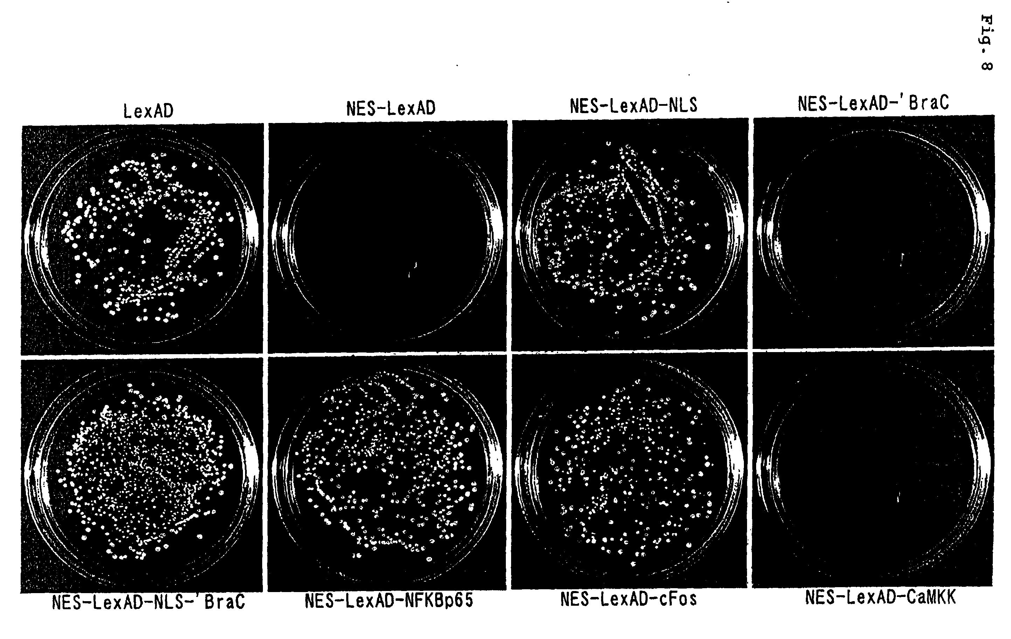

Each plasmid was introduced into the EGY48 strain, and the expression of reporter gene LEU2 was observed. Specifically, the yeast strain was transformed with the various plasmids mentioned in (1) and directly plated onto SD media (-HIS, -LEU). Figure 8 shows the results of cultivation at 30°C for three days. A colony formed in LexAD, which has no NES, probably because of passive diffusion into the nucleus. In contrast, colony formation was completely suppressed in NES-LexAD, where NES had been introduced. However, a colony did form in NES-LexAD-NLS, where NLS had been introduced. Similarly, colony formation was observed in NES-LexAD-NLS-'BraC, NES-LexAD-NFKBp65, and NES-LexAD-cFos that had typical NLS. In contrast, colony formation was completely suppressed in NES-LexAD-'BraC and NES-LexAD-CaMKK, which had no nuclear transportability. These results demonstrate that the system using the pNS vector can specifically detect a cDNA fragment with nuclear transportability.

### Industrial Applicability

The present invention enables easy determination of whether a peptide encoded by a test DNA has nuclear transportability or not by using the expression of a reporter gene as an indication. The present invention also enables quick, efficient and systematic cloning of a DNA encoding a protein with nuclear transportability by detecting the expression of a reporter gene as an indication. Furthermore, the present invention not only promotes obtaining a DNA encoding a novel intranuclear protein with biologically important function extremely well but also provides gene expression information (stage, position, expression frequency, etc.) that is very useful for studying the function of intranuclear proteins. The use of the information is expected to contribute significantly to the development of epoch-making pharmaceuticals.

Sequence Listing
(1) Name of Applicant: Helix Research Institute
(2) Title of the Invention: Method for Detecting and Isolating Nuclear Transport
Protein
(3) Reference Number: H1-804DP1PCT
(4) Application Number:
(5) Filing Date:
(6) Country where the priority application was filed and the application number
of the application:
Japan, No. Hei 9-124795
Japan, No. Hei 9-309686
(7) Priority date: April 28, 1998 and October 24, 1998
(8) Number of Sequences: 26

SEQ ID NO: 1:
SEQUENCE LENGTH: 30
SEQUENCE TYPE: nucleic acid
STRANDEDNESSS: single
TOPOLOGY: linear
MOLECULE TYPE: other nucleic acid, synthetic DNA
SEQUENCE DESCRIPTION: SEQ ID NO: 1
TTTGAATTCG CCAATTTTAA TCAAAGTGGG 30

SEQ ID NO: 2:
SEQUENCE LENGTH: 32
SEQUENCE TYPE: nucleic acid
STRANDEDNESSS: single
TOPOLOGY: linear
MOLECULE TYPE: other nucleic acid, synthetic DNA
SEQUENCE DESCRIPTION: SEQ ID NO: 2
TAGCATCTAT GACTTTTTGG GGCGTTCAAG TG 32

SEQ ID NO: 3:

SEQUENCE LENGTH: 342
SEQUENCE TYPE: nucleic acid
STRANDEDNESSS: single
TOPOLOGY: linear
MOLECULE TYPE: cDNA to mRNA
FEATURE:
NAME/KEY: domain
LOCATION: 1..342
IDENTIFICATION METHOD: S
SEQUENCE DESCRIPTION: SEQ ID NO: 3
GCC AAT TTT AAT CAA AGT GGG AAT ATT GCT GAT AGC TCA TTG TCC TTC 48
Ala Asn Phe Asn Gln Ser Gly Asn Ile Ala Asp Ser Ser Leu Ser Phe
1               5                   10                  15
ACT TTC ACT AAC AGT AGC AAC GGT CCG AAC CTC ATA ACA ACT CAA ACA 96
Thr Phe Thr Asn Ser Ser Asn Gly Pro Asn Leu Ile Thr Thr Gln Thr
            20                  25                  30
AAT TCT CAA GCG CTT TCA CAA CCA ATT GCC TCC TCT AAC GTT CAT GAT 144
Asn Ser Gln Ala Leu Ser Gln Pro Ile Ala Ser Ser Asn Val His Asp
        35                  40                  45
AAC TTC ATG AAT AAT GAA ATC ACG GCT AGT AAA ATT GAT GAT GGT AAT 192
Asn Phe Met Asn Asn Glu Ile Thr Ala Ser Lys Ile Asp Asp Gly Asn
    50                  55                  60
AAT TCA AAA CCA CTG TCA CCT GGT TGG ACG GAC CAA ACT GCG TAT AAC 240
Asn Ser Lys Pro Leu Ser Pro Gly Trp Thr Asp Gln Thr Ala Tyr Asn
65                  70                  75                  80
GCG TTT GGA ATC ACT ACA GGG ATG TTT AAT ACC ACT ACA ATG GAT GAT 288
Ala Phe Gly Ile Thr Thr Gly Met Phe Asn Thr Thr Thr Met Asp Asp
                85                  90                  95
GTA TAT AAC TAT CTA TTC GAT GAT GAA GAT ACC CCA CCA AAC CCA AAA 336
Val Tyr Asn Tyr Leu Phe Asp Asp Glu Asp Thr Pro Pro Asn Pro Lys
            100                 105                 110
AAA GAG 342
Lys Glu

SEQ ID NO: 4:
SEQUENCE LENGTH: 609
SEQUENCE TYPE: nucleic acid

STRANDEDNESSS: double
TOPOLOGY: linear
MOLECULE TYPE: cDNA to mRNA
FEATURE:
NAME/KEY: CDS
LOCATION: 1..606
IDENTIFICATION METHOD: S
SEQUENCE DESCRIPTION: SEQ ID NO: 4
ATG AAA GCG TTA ACG GCC AGG CAA CAA GAG GTG TTT GAT CTC ATC CGT 48
Met Lys Ala Leu Thr Ala Arg Gln Gln Glu Val Phe Asp Leu Ile Arg
1               5                   10                  15

GAT CAC ATC AGC CAG ACA GGT ATG CCG CCG ACG CGT GCG GAA ATC GCG 96
Asp His Ile Ser Gln Thr Gly Met Pro Pro Thr Arg Ala Glu Ile Ala
            20                  25                  30

CAG CGT TTG GGG TTC CGT TCC CCA AAC GCG GCT GAA GAA CAT CTG AAG 144
Gln Arg Leu Gly Phe Arg Ser Pro Asn Ala Ala Glu Glu His Leu Lys
        35                  40                  45
GCG CTG GCA CGC AAA GGC GTT ATT GAA ATT GTT TCC GGC GCA TCA CGC 192
Ala Leu Ala Arg Lys Gly Val Ile Glu Ile Val Ser Gly Ala Ser Arg
    50                  55                  60
GGG ATT CGT CTG TTG CAG GAA GAG GAA GAA GGG TTG CCG CTG GTA GGT 240
Gly Ile Arg Leu Leu Gln Glu Glu Glu Glu Gly Leu Pro Leu Val Gly
65                  70                  75                  80
CGT GTG GCT GCC GGT GAA CCA CTT CTG GCG CAA CAG CAT ATT GAA GGT 288
Arg Val Ala Ala Gly Glu Pro Leu Leu Ala Gln Gln His Ile Glu Gly
                85                  90                  95
CAT TAT CAG GTC GAT CCT TCC TTA TTC AAG CCG AAT GCT GAT TTC CTG 336
His Tyr Gln Val Asp Pro Ser Leu Phe Lys Pro Asn Ala Asp Phe Leu
            100                 105                 110
CTG CGC GTC AGC GGG ATG TCG ATG AAA GAT ATC GGC ATT ATG GAT GGT 384
Leu Arg Val Ser Gly Met Ser Met Lys Asp Ile Gly Ile Met Asp Gly
        115                 120                 125
GAC TTG CTG GCA GTG CAT AAA ACT CAG GAT GTA CGT AAC GGT CAG GTC 432
Asp Leu Leu Ala Val His Lys Thr Gln Asp Val Arg Asn Gly Gln Val
    130                 135                 140

GTT GTC GCA CGT ATT GAT GAC GAA GTT ACC GTT AAG CGC CTG AAA AAA 480
Val Val Ala Arg Ile Asp Asp Glu Val Thr Val Lys Arg Leu Lys Lys
145                 150                 155                 160
CAG GGC AAT AAA GTC GAA CTG TTG CCA GAA AAT AGC GAG TTT AAA CCA 528
Gln Gly Asn Lys Val Glu Leu Leu Pro Glu Asn Ser Glu Phe Lys Pro
                165                 170                 175
ATT GTC GTT GAC CTT CGT CAG CAG AGC TTC ACC ATT GAA GGG CTG GCG 576
Ile Val Val Asp Leu Arg Gln Gln Ser Phe Thr Ile Glu Gly Leu Ala
            180                 185                 190
GTT GGG GTT ATT CGC AAC GGC GAC TGG CTG TAA 609
Val Gly Val Ile Arg Asn Gly Asp Trp Leu
        195                 200

SEQ ID NO: 5:
SEQUENCE LENGTH: 10
SEQUENCE TYPE: amino acid
TOPOLOGY: linear
MOLECULE TYPE: peptide
SEQUENCE DESCRIPTION: SEQ ID NO: 5
Gln Leu Pro Pro Leu Glu Arg Leu Thr Leu
1               5                   10

SEQ ID NO: 6:
SEQUENCE LENGTH: 30
SEQUENCE TYPE: nucleic acid
STRANDEDNESSS: single
TOPOLOGY: linear
MOLECULE TYPE: other nucleic acid, synthetic DNA
SEQUENCE DESCRIPTION: SEQ ID NO: 6
ACAGCTGCCA CCGATTGAGA GACTTACGTT 30

SEQ ID NO: 7:
SEQUENCE LENGTH: 30
SEQUENCE TYPE: nucleic acid
STRANDEDNESSS: single
TOPOLOGY: linear
MOLECULE TYPE: other nucleic acid, synthetic DNA

SEQUENCE DESCRIPTION: SEQ ID NO: 7
TGTCGACGGT GGCTAACTCT CTGAATGCAA 30

SEQ ID NO: 8:
SEQUENCE LENGTH: 1080
SEQUENCE TYPE: nucleic acid
STRANDEDNESSS: double
TOPOLOGY: linear
MOLECULE TYPE: cDNA to mRNA
FEATURE:
NAME/KEY: CDS
LOCATION: 1..1077
IDENTIFICATION METHOD: E
SEQUENCE DESCRIPTION: SEQ ID NO: 8
ATG AAA GCG TTA CAG CTG CCA CCG ATT GAG AGA CTT ACG TTA ACG GCC 48
Met Lys Ala Leu Gln Leu Pro Pro Ile Glu Arg Leu Thr Leu Thr Ala
1               5                   10                  15
AGG CAA CAA GAG GTG TTT GAT CTC ATC CGT GAT CAC ATC AGC CAG ACA 96
Arg Gln Gln Glu Val Phe Asp Leu Ile Arg Asp His Ile Ser Gln Thr
            20                  25                  30
GGT ATG CCG CCG ACG CGT GCG GAA ATC GCG CAG CGT TTG GGG TTC CGT 144
Gly Met Pro Pro Thr Arg Ala Glu Ile Ala Gln Arg Leu Gly Phe Arg
        35                  40                  45
TCC CCA AAC GCG GCT GAA GAA CAT CTG AAG GCG CTG GCA CGC AAA GGC 192
Ser Pro Asn Ala Ala Glu Glu His Leu Lys Ala Leu Ala Arg Lys Gly
    50                  55                  60
GTT ATT GAA ATT GTT TCC GGC GCA TCA CGC GGG ATT CGT CTG TTG CAG 240
Val Ile Glu Ile Val Ser Gly Ala Ser Arg Gly Ile Arg Leu Leu Gln
65                  70                  75                  80
GAA GAG GAA GAA GGG TTG CCG CTG GTA GGT CGT GTG GCT GCC GGT GAA 288
Glu Glu Glu Glu Gly Leu Pro Leu Val Gly Arg Val Ala Ala Gly Glu
                85                  90                  95
CCA CTT CTG GCG CAA CAG CAT ATT GAA GGT CAT TAT CAG GTC GAT CCT 336
Pro Leu Leu Ala Gln Gln His Ile Glu Gly His Tyr Gln Val Asp Pro
            100                 105                 110
TCC TTA TTC AAG CCG AAT GCT GAT TTC CTG CTG CGC GTC AGC GGG ATG 384
Ser Leu Phe Lys Pro Asn Ala Asp Phe Leu Leu Arg Val Ser Gly Met

        115                 120                 125
TCG ATG AAA GAT ATC GGC ATT ATG GAT GGT GAC TTG CTG GCA GTG CAT 432
Ser Met Lys Asp Ile Gly Ile Met Asp Gly Asp Leu Leu Ala Val His
    130                 135                 140
AAA ACT CAG GAT GTA CGT AAC GGT CAG GTC GTT GTC GCA CGT ATT GAT 480
Lys Thr Gln Asp Val Arg Asn Gly Gln Val Val Val Ala Arg Ile Asp
145                 150                 155                 160
GAC GAA GTT ACC GTT AAG CGC CTG AAA AAA CAG GGC AAT AAA GTC GAA 528
Asp Glu Val Thr Val Lys Arg Leu Lys Lys Gln Gly Asn Lys Val Glu
                165                 170                 175
CTG TTG CCA GAA AAT AGC GAG TTT AAA CCA ATT GTC GTT GAC CTT CGT 576
Leu Leu Pro Glu Asn Ser Glu Phe Lys Pro Ile Val Val Asp Leu Arg
            180                 185                 190
CAG CAG AGC TTC ACC ATT GAA GGG CTG GCG GTT GGG GTT ATT CGC AAC 624
Gln Gln Ser Phe Thr Ile Glu Gly Leu Ala Val Gly Val Ile Arg Asn
        195                 200                 205
GGC GAC TGG CTG GAA TTC GCC AAT TTT AAT CAA AGT GGG AAT ATT GCT 672
Gly Asp Trp Leu Glu Phe Ala Asn Phe Asn Gln Ser Gly Asn Ile Ala
    210                 215                 220
GAT AGC TCA TTG TCC TTC ACT TTC ACT AAC AGT AGC AAC GT CCG AAC 720
Asp Ser Ser Leu Ser Phe Thr Phe Thr Asn Ser Ser Asn Gly Pro Asn
225                 230                 235                 240
CTC ATA ACA ACT CAA ACA AAT TCT CAA GCG CTT TCA CAA CCA ATT GCC 768
Leu Ile Thr Thr Gln Thr Asn Ser Gln Ala Leu Ser Gln Pro Ile Ala
                245                 250                 255
TCC TCT AAC GTT CAT GAT AAC TTC ATG AAT AAT GAA ATC ACG GCT AGT 816
Ser Ser Asn Val His Asp Asn Phe Met Asn Asn Glu Ile Thr Ala Ser
            260                 265                 270
AAA ATT GAT GAT GGT AAT AAT TCA AAA CCA CTG TCA CCT GGT TGG ACG 864
Lys Ile Asp Asp Gly Asn Asn Ser Lys Pro Leu Ser Pro Gly Trp Thr
        275                 280                 285
GAC CAA ACT GCG TAT AAC GCG TTT GGA ATC ACT ACA GGG ATG TTT AAT 912
Asp Gln Thr Ala Tyr Asn Ala Phe Gly Ile Thr Thr Gly Met Phe Asn
    290                 295                 300
ACC ACT ACA ATG GAT GAT GTA TAT AAC TAT CTA TTC GAT GAT GAA GAT 960
Thr Thr Thr Met Asp Asp Val Tyr Asn Tyr Leu Phe Asp Asp Glu Asp
305                 310                 315                 320

ACC CCA CCA AAC CCA AAA AAA GAG ATC TCT ATG GCT TAC CCA TAC GAT 1008
Thr Pro Pro Asn Pro Lys Lys Glu Ile Ser Met Ala Tyr Pro Tyr Asp
                325                 330                 335
GTT CCA GAT TAC GCT AGC TTG GGT GGT CAT ATG GCC ATG GCG GCC GCT 1056
Val Pro Asp Tyr Ala Ser Leu Gly Gly His Met Ala Met Ala Ala Ala
            340                 345                 350
CGA GTC GAC CTG CAG CCA AGC TAA 1080
Arg Val Asp Leu Gln Pro Ser
        355

SEQ ID NO: 9:
SEQUENCE LENGTH: 1152
SEQUENCE TYPE: nucleic acid
STRANDEDNESSS: double
TOPOLOGY: linear
MOLECULE TYPE: cDNA to mRNA
FEATURE:
NAME/KEY: CDS
LOCATION: 1..1149
IDENTIFICATION METHOD: S
SEQUENCE DESCRIPTION: SEQ ID NO: 9
ATG GCT AAG ATC TCT CCC GGG CTC GAG CTC ATG AAG AAG GGT ACT CAG 48
Met Ala Lys Ile Ser Pro Gly Leu Glu Leu Met Lys Lys Gly Thr Gln
1               5                   10                  15
CGT CTA TCC CGC CTG TTC GCC GCG ATG GCC ATT GCC GGG TTC GCC AGC 96
Arg Leu Ser Arg Leu Phe Ala Ala Met Ala Ile Ala Gly Phe Ala Ser
            20                  25                  30
TAC TCC ATG GCC GCC GAC ACC ATC AAG ATC GCC CTG GCT GGC CCG GTC 144
Tyr Ser Met Ala Ala Asp Thr Ile Lys Ile Ala Leu Ala Gly Pro Val
        35                  40                  45
ACC GGT CCG GTA GCC CAG TAC GGC GAC ATG CAG CGC GCC GGT GCG CTG 192
Thr Gly Pro Val Ala Gln Tyr Gly Asp Met Gln Arg Ala Gly Ala Leu
    50                  55                  60
ATG GCA ATC GAA CAG ATC AAC AAG GCA GGC GGC GTG AAC GGC GCG CAA 240
Met Ala Ile Glu Gln Ile Asn Lys Ala Gly Gly Val Asn Gly Ala Gln
65                  70                  75                  80
CTC GAA GGC GTG ATC TAC GAC GAC GCC TGC GAT CCC AAG CAG GCC GTG 288

Leu Glu Gly Val Ile Tyr Asp Asp Ala Cys Asp Pro Lys Gln Ala Val
                85                  90                  95
GCG GTC GCC AAC AAG GTG GTC AAC GAC GGC GTC AAG TTC GTG GTC GGT     336
Ala Val Ala Asn Lys Val Val Asn Asp Gly Val Lys Phe Val Val Gly
            100                 105                 110
CAT GTC TGC TCC AGC TCC ACC CAA CCC GCC ACC GAC ATC TAC GAA GAC     384
His Val Cys Ser Ser Ser Thr Gln Pro Ala Thr Asp Ile Tyr Glu Asp
        115                 120                 125
GAA GGC GTG CTG ATG ATC ACC CCG TCG GCC ACC GCC CCG GAA ATC ACC     432
Glu Gly Val Leu Met Ile Thr Pro Ser Ala Thr Ala Pro Glu Ile Thr
    130                 135                 140
TCG CGC GGC TAC AAG CTG ATC TTC CGC ACC ATC GGC CTG GAC AAC ATG     480
Ser Arg Gly Tyr Lys Leu Ile Phe Arg Thr Ile Gly Leu Asp Asn Met
145                 150                 155                 160
CAG GGC CCG GTG GCC GGC AAG TTC ATC GCC GAA CGC TAC AAG GAC AAG     528
Gln Gly Pro Val Ala Gly Lys Phe Ile Ala Glu Arg Tyr Lys Asp Lys
                165                 170                 175
ACC ATC GCG GTA CTG CAC GAC AAG CAG CAG TAC GGC GAA GGC ATC GCC     576
Thr Ile Ala Val Leu His Asp Lys Gln Gln Tyr Gly Glu Gly Ile Ala
            180                 185                 190
ACC GAG GTG AAG AAG ACC GTG GAA GAC GCC GGC ATC AAG GTT GCC GTC     624
Thr Glu Val Lys Lys Thr Val Glu Asp Ala Gly Ile Lys Val Ala Val
        195                 200                 205
TTC GAA GGC CTG AAC GCC GGC GAC AAG GAC TTC AAC GCG CTG ATC AGC     672
Phe Glu Gly Leu Asn Ala Gly Asp Lys Asp Phe Asn Ala Leu Ile Ser
    210                 215                 220
AAG CTG AAG AAA GCC GGC GTG CAG TTC GTC TAC TTC GGC GGC TAC CAC     720
Lys Leu Lys Lys Ala Gly Val Gln Phe Val Tyr Phe Gly Gly Tyr His
225                 230                 235                 240
CCA GAA ATG GGC CTG CTG CTG CGC CAG GCC AAG CAG GCC GGG CTG GAC     768
Pro Glu Met Gly Leu Leu Leu Arg Gln Ala Lys Gln Ala Gly Leu Asp
                245                 250                 255
GCG CGC TTC ATG GGC CCG GAA GGG GTC GGC AAC AGC GAA ATC ACC GCG     816
Ala Arg Phe Met Gly Pro Glu Gly Val Gly Asn Ser Glu Ile Thr Ala
            260                 265                 270
ATC GCC GGC GAC GCT TCG GAA GGC ATG CTG GCG ACC CTG CCG CGC GCC     864
Ile Ala Gly Asp Ala Ser Glu Gly Met Leu Ala Thr Leu Pro Arg Ala

        275                 280                 285
TTC GAG CAG GAT CCG AAG AAC AAG GCC CTG ATC GAC GCC TTC AAG GCG 912
Phe Glu Gln Asp Pro Lys Asn Lys Ala Leu Ile Asp Ala Phe Lys Ala
    290                 295                 300
AAG AAC CAG GAT CCG AGC GGC ATC TTC GTC CTG CCC GCC TAC TCC GCG 960
Lys Asn Gln Asp Pro Ser Gly Ile Phe Val Leu Pro Ala Tyr Ser Ala
305                 310                 315                 320
GTC ACA GTG ATC GCC AAG GGC ATC GAG AAA GCC GGC GAG GCC GAT CCG 1008
Val Thr Val Ile Ala Lys Gly Ile Glu Lys Ala Gly Glu Ala Asp Pro
                325                 330                 335
GAG AAG GTC GCC GAG GCC CTG CGC GCC AAC ACC TTC GAG ACT CCC ACC 1056
Glu Lys Val Ala Glu Ala Leu Arg Ala Asn Thr Phe Glu Thr Pro Thr
            340                 345                 350
GGG AAC CTC GGG TTC GAC GAG AAG GGC GAC CTG AAG AAC TTC GAC TTC 1104
Gly Asn Leu Gly Phe Asp Glu Lys Gly Asp Leu Lys Asn Phe Asp Phe
        355                 360                 365
ACC GTC TAC GAG TGG CAC AAG GAC GCC ACC CGG ACC GAG GTC AAG 1149
Thr Val Tyr Glu Trp His Lys Asp Ala Thr Arg Thr Glu Val Lys
    370                 375                 380
TAA 1152

SEQ ID NO: 10:
SEQUENCE LENGTH: 12
SEQUENCE TYPE: amino acid
TOPOLOGY: linear
MOLECULE TYPE: peptide
SEQUENCE DESCRIPTION: SEQ ID NO: 10
Ser Glu Pro Pro Lys Lys Lys Arg Lys Val Glu Thr
1               5                   10

SEQ ID NO: 11:
SEQUENCE LENGTH: 37
SEQUENCE TYPE: nucleic acid
STRANDEDNESSS: single
TOPOLOGY: linear
MOLECULE TYPE: other nucleic acid, synthetic DNA
SEQUENCE DESCRIPTION: SEQ ID NO: 11

CTAGCGAGCC TCCAAAAAAG AAGAGAAAGG TCGAAAC 37

SEQ ID NO: 12:
SEQUENCE LENGTH: 37
SEQUENCE TYPE: nucleic acid
STRANDEDNESSS: single
TOPOLOGY: linear
MOLECULE TYPE: other nucleic acid, synthetic DNA
SEQUENCE DESCRIPTION: SEQ ID NO: 12
GCTCGGAGGT TTTTTCTTCT CTTTCCAGCT TTGGTAC 37

SEQ ID NO: 13:
SEQUENCE LENGTH: 419
SEQUENCE TYPE: nucleic acid
STRANDEDNESSS: double
TOPOLOGY: linear
MOLECULE TYPE: genomic DNA
SEQUENCE DESCRIPTION: SEQ ID NO: 13
TCGACTGCTG TATATAAAAC CAGTGGTTAT ATGTACAGTA CTGCTGTATA TAAAACCAGT 60
GGTTATATGT ACAGTACGTC GAGGGAATCA AATTAACAAC CATAGGATGA TAATGCGATT 120
AGTTTTTTAG CCTTATTTCT GGGGTAATTA ATCAGCGAAG CGATGATTTT TGATCTATTA 180
ACAGATATAT AAATGCAAAA ACTGCATAAC CACTTTAACT AATACTTTCA ACATTTTCGG 240
TTTGTATTAC TTCTTATTCA AATGTAATAA AAGTATCAAC AAAAAATTGT TAATATACCT 300
CTATACTTTA ACGTCAAGGA GAAAAAACTA TAATGACTAA ATCTCATTCA GAAGAAGTGA 360
TTGTACCTGA GTTCAATTCT AGCGCAAAGG AATTACCAAG ACCATTGGCC GAAAAGTGC 419

SEQ ID NO: 14:
SEQUENCE LENGTH: 12
SEQUENCE TYPE: nucleic acid
STRANDEDNESSS: single
TOPOLOGY: linear
MOLECULE TYPE: other nucleic acid, synthetic DNA
SEQUENCE DESCRIPTION: SEQ ID NO: 14
AATTGACCAC CC 12

SEQ ID NO: 15:
SEQUENCE LENGTH: 12

SEQUENCE TYPE: nucleic acid
STRANDEDNESSS: single
TOPOLOGY: linear
MOLECULE TYPE: other nucleic acid, synthetic DNA
SEQUENCE DESCRIPTION: SEQ ID NO: 15
CTGGTGGGTT AA 12

SEQ ID NO: 16:
SEQUENCE LENGTH: 25
SEQUENCE TYPE: nucleic acid
STRANDEDNESSS: single
TOPOLOGY: linear
MOLECULE TYPE: other nucleic acid, synthetic DNA
SEQUENCE DESCRIPTION: SEQ ID NO: 16
CTAGCTTGGG TGGAATTCAT ATGGC 25

SEQ ID NO: 17:
SEQUENCE LENGTH: 24
SEQUENCE TYPE: nucleic acid
STRANDEDNESSS: single
TOPOLOGY: linear
MOLECULE TYPE: other nucleic acid, synthetic DNA
SEQUENCE DESCRIPTION: SEQ ID NO: 17
GAACCCACCT TAAGTATACG GTAC 24

SEQ ID NO: 18:
SEQUENCE LENGTH: 11
SEQUENCE TYPE: nucleic acid
STRANDEDNESSS: single
TOPOLOGY: linear
MOLECULE TYPE: other nucleic acid, synthetic DNA
SEQUENCE DESCRIPTION: SEQ ID NO: 18
CTGCATGCAC C 11

SEQ ID NO: 19:
SEQUENCE LENGTH: 14
SEQUENCE TYPE: nucleic acid

STRANDEDNESSS: single
TOPOLOGY: linear
MOLECULE TYPE: other nucleic acid, synthetic DNA
SEQUENCE DESCRIPTION: SEQ ID NO: 19
ATGGACGTAC GTGG 14

SEQ ID NO: 20:
SEQUENCE LENGTH: 32
SEQUENCE TYPE: nucleic acid
STRANDEDNESSS: single
TOPOLOGY: linear
MOLECULE TYPE: other nucleic acid, synthetic DNA
SEQUENCE DESCRIPTION: SEQ ID NO: 20
CTATTCGATG ATGAAGATAC CCCACCAAAC CC 32

SEQ ID NO: 21:
SEQUENCE LENGTH: 30
SEQUENCE TYPE: nucleic acid
STRANDEDNESSS: single
TOPOLOGY: linear
MOLECULE TYPE: other nucleic acid, synthetic DNA
SEQUENCE DESCRIPTION: SEQ ID NO: 20
GAAATTCGCC CGGAATTAGC TTGGCTGCAG 30

SEQ ID NO: 22:
SEQUENCE LENGTH: 32
SEQUENCE TYPE: nucleic acid
STRANDEDNESSS: single
TOPOLOGY: linear
MOLECULE TYPE: other nucleic acid, synthetic DNA
SEQUENCE DESCRIPTION: SEQ ID NO: 22
CTATTCGATG ATGAAGATAC CCCACCAAAC CC 32

SEQ ID NO: 23:
SEQUENCE LENGTH: 30
SEQUENCE TYPE: nucleic acid
STRANDEDNESSS: single

TOPOLOGY: linear
MOLECULE TYPE: other nucleic acid, synthetic DNA
SEQUENCE DESCRIPTION: SEQ ID NO: 23
GAAATTCGCC CGGAATTAGC TTGGCTGCAG 30

SEQ ID NO: 24:
SEQUENCE LENGTH: 32
SEQUENCE TYPE: nucleic acid
STRANDEDNESSS: single
TOPOLOGY: linear
MOLECULE TYPE: other nucleic acid, synthetic DNA
SEQUENCE DESCRIPTION: SEQ ID NO: 24
TTTCAATTGG AATGGACGAA CTGTTCCCCC TC 32

SEQ ID NO: 25:
SEQUENCE LENGTH: 35
SEQUENCE TYPE: nucleic acid
STRANDEDNESSS: single
TOPOLOGY: linear
MOLECULE TYPE: other nucleic acid, synthetic DNA
SEQUENCE DESCRIPTION: SEQ ID NO: 25
GCGCAGCGAG TCAGTGAGCG AGGAAGCGGA AGAGG 35

SEQ ID NO: 26:
SEQUENCE LENGTH: 35
SEQUENCE TYPE: nucleic acid
STRANDEDNESSS: single
TOPOLOGY: linear
MOLECULE TYPE: other nucleic acid, synthetic DNA
SEQUENCE DESCRIPTION: SEQ ID NO: 26
TTTGAATTCT AATGATGTTC TCGGGTTTCA ACGCG 35

## Claims

1. A method for detecting nuclear transportability of a peptide encoded by a test DNA, the method comprising introducing a fusion DNA formed by a DNA encoding a transcription factor without nuclear transportability and the test DNA into an eukaryotic host having in its nucleus a promoter region that is activated when said transcription factor binds thereto and a reporter gene connected to the downstream of said promoter region, and detecting expression of said reporter gene.

2. The method of claim 1, wherein the transcription factor without nuclear transportability is a fusion protein comprising a nuclear export signal, a DNA binding domain, and a transcription activating domain.

3. The method of claim 1, wherein the transcription factor without nuclear transportability is a fusion protein comprising a nuclear export signal, a LexA protein, and a GAL4-transcription activating domain, and the promoter region activated when said transcription factor binds thereto is that of a GAL1 gene whose operator sequence is replaced with that of LexA.

4. The method of claim 3, wherein the nuclear export signal is a peptide comprising the amino acid sequence set forth in SEQ ID NO; 5.

5. The method of any one of claims 1 to 4, wherein the reporter gene is LEU2 and/or a β-galactosidase gene.

6. A method for isolating a DNA encoding a peptide with nuclear transportability, the method comprising introducing a fusion DNA formed by a DNA encoding a transcription factor without nuclear transportability and a test DNA into a eukaryotic host having in its nucleus a promoter region activated when said transcription factor binds thereto and a reporter gene connected downstream of said promoter region, detecting the expression of said reporter gene, and isolating the test DNA from the eukaryotic host in which the expression has been detected.

7. The method of claim 6, wherein the transcription factor without nuclear transportability is a fusion protein comprising a nuclear export signal, a DNA binding domain, and a transcription activating domain.

8. The method of claim 6, wherein the transcription factor without nuclear transportability is a fusion protein comprising a nuclear export signal, a LexA protein, and a GAL4-transcription activating domain, and the promoter region activated when said transcription factor binds thereto is that of a GAL1 gene whose operator sequence is replaced with that of LexA.

9. The method of claim 8, wherein the nuclear export signal is a peptide comprising the amino acid sequence set forth in SEQ ID NO: 5.

10. The method of any one of claims 6 to 9, wherein the reporter gene is LEU2 and/or a β-galactosidase gene.

11. A vector comprising a DNA encoding a transcription factor without nuclear transportability and an introduction site for a test DNA adjacent thereto.

12. The vector of claim 11, wherein the transcription factor without nuclear transportability is a fusion protein comprising a nuclear export signal, a DNA binding domain, and a transcription activating domain.

13. The vector of claim 11, wherein the transcription factor without nuclear transportability is a fusion protein comprising a nuclear export signal, a LexA protein, and a GAL4-transcription activating domain.

14. The vector of claim 13, wherein the nuclear export signal is the peptide comprising the amino acid sequence set forth in SEQ ID NO: 5.

15. A kit comprising (i) a vector comprising a DNA encoding a transcription factor without nuclear transportability and an introduction site for a test DNA adjacent thereto, and (ii) a eukaryotic host having in its nucleus an expression unit comprising a promoter region activated when said transcription factor binds thereto and a reporter gene connected to the downstream of said promoter region.

16. The kit of claim 15, wherein the transcription factor without nuclear transportability is a fusion protein comprising a nuclear export signal, a DNA binding domain, and a transcription activating domain.

17. The method of claim 15, wherein the transcription factor without nuclear transportability is a fusion protein comprising a nuclear export signal, a LexA protein, and a GAL4-transcription activating domain, and the promoter region activated when said transcription factor binds thereto is that of a GAL1 gene whose operator sequence is replaced with that of LexA, and the eukaryotic host is yeast.

18. The kit of claim 17, wherein the nuclear export signal is a peptide comprising the amino acid sequence set forth in SEQ ID NO: 5.

19. The kit of any one of claims 15 to 18, wherein the reporter gene is LEU2 and/or a β-galactosidase gene.
